# EUROPÄISCHE PATENTSCHRIFT

(11) **EP 1 451 224 B1**
(45) Veröffentlichungstag und Bekanntmachung des Hinweises auf die Patenterteilung: **15.08.2012**
(21) Anmeldenummer: 02804143.2
(22) Anmeldetag: 29.10.2002
(51) Int. Cl.: C07K 14/705

(54) **PEPTID ODER PROTEIN ENTHALTEND EIN C'-D LOOP DER CD28 REZEPTORFAMILIE**
PEPTIDE OR PROTEIN CONTAINING A C'-D LOOP OF THE CD28 RECEPTOR FAMILY
PEPTIDE OU PROTEINE CONTENANT UNE BOUCLE C'-D DE LA FAMILLE DE RECEPTEURS CD28

(30) Priorität: 04.12.2001 DE 10160516; 10.01.2002 DE 10200714
(43) Veröffentlichungstag der Anmeldung: 01.09.2004
(73) Patentinhaber: TheraMAB LLC, Moscow 117246 (RU)
(72) Erfinder: HÜNIG, Thomas, 97076 Würzburg (DE); LÜHDER, Fred c/oInst. für mult. Sklerose Forschung, 37073 Göttingen (DE); HANKE, Thomas, 97074 Würzburg (DE)
(74) Vertreter: Jungblut, Bernhard Jakob
(86) Internationale Anmeldenummer: PCT/DE2002/004064
(87) Internationale Veröffentlichungsnummer: WO 2003/048194

(56) Entgegenhaltungen:
- EP-A- 0 947 582
- WO-A-01/71042
- NIERMAN WILLIAM C ET AL: "Complete genome sequence of Caulobacter crescentus." PROCEEDINGS OF THE NATIONAL ACADEMY OF SCIENCES OF THE UNITED STATES, Bd. 98, Nr. 7, 27. März 2001 (2001-03-27), Seiten 4136-4141, XP002250580 March 27, 2001 & DATABASE EBI

## Beschreibung

### Gebiet der Erfindung.

Die Erfindung betrifft ein Protein oder Peptid enthaltend eine Teilsequenz eines Mitgliedes der CD28 Familie, eine Nukleinsäure codierend für ein solches Peptid, ein Plasmid enthaltend eine solche Nukleinsäure, Hybridomzellen, welche monoklonale Antikörper (mAb) bilden, die an ein solches Peptid binden, aus solchen Hybridomzellen erhältliche mAb sowie Verwendungen des Peptids und der mAb.

### Definitionen.

Als monoklonale Antikörper sind Antikörper bezeichnet, die von Hybrid-Zellinien (sog. Hybridomen) produziert werden, die typischerweise durch Fusion einer Antikörper produzierenden B-Zelle tierischer oder menschlicher Herkunft mit einer geeigneten Myelom Tumorzelle entstanden sind.

Die Aminosäuresequenz von Human CD28 ist unter der Accession No. NM_006139 bekannt.

Die Aminosäuresequenz von Human CTLA-4 ist unter der Accession No. L15006 bekannt.

Die Aminosäuresequenz von Human ICOS ist unter der Accession No. AJ277832 bekannt.

Die Aminosäuresequenz von Human PD-1 ist unter der Accession No. U64863 bekannt.

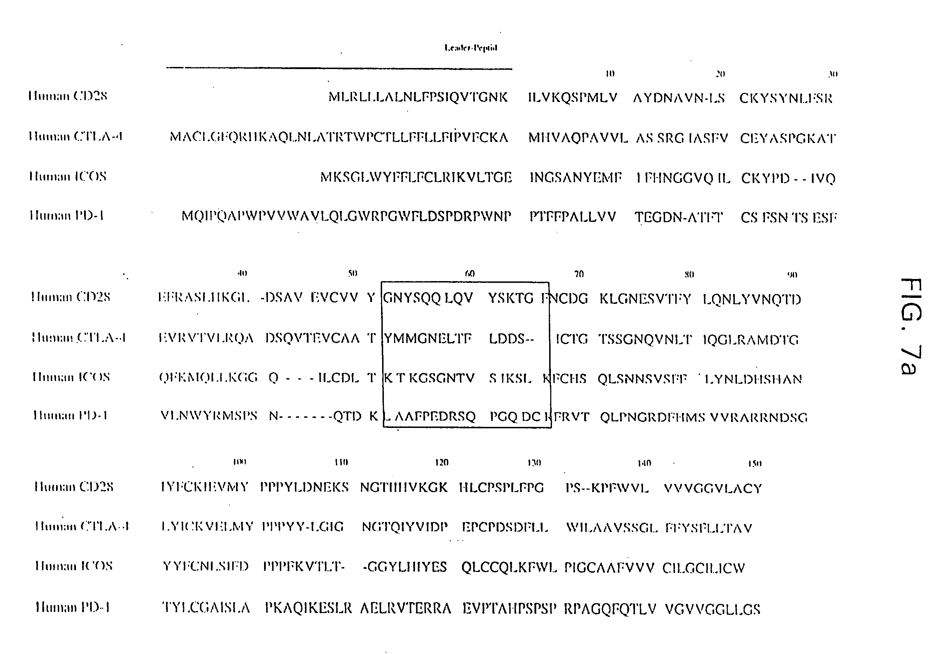

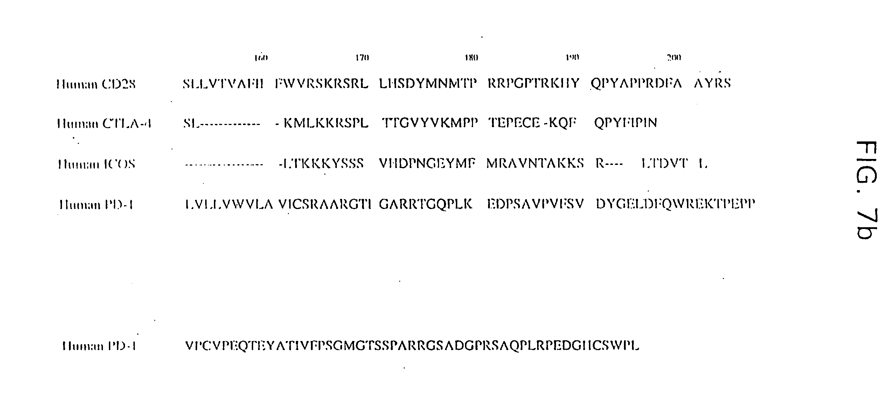

Das C'-D Loop von CD28 umfaßt die Aminosäuren 52 bis 66 der vorstehenden CD28 Sequenz (Numerierung gemäß Fig. 7, siehe auch Ostrov, D.A., et al.; Science (2000), 290:816-819). Unter dem Begriff des C'-D Loops sollen im Folgenden auch beliebige Teilsequenzen hieraus verstanden sein.

Ein Loop bzw. eine darin angeordnete Bindungsstelle ist frei zugänglich, wenn für einen definierten Bindungspartner für die Bindungsstelle im Loop keine sterische Hinderung durch an das Loop anschließende Sequenzen oder Moleküle vorliegt.

Mit Aktivierung von T-Lymphozyten ist die Vermehrung der Stoffwechselaktivität, Vergrößerung des Zellvolumens, Synthese immunologisch wichtiger Moleküle und Eintritt in die Zellteilung (Proliferation) von T-Lymphozyten auf einen äußeren Reiz hin bezeichnet. Inhibierung ist der hierzu entgegengesetzte Prozeß. Beispielsweise werden solche Vorgänge durch Besetzung des CD28-Moleküls auf T-Zellen durch besondere CD28-spezifische monoklonale Antikörper ausgelöst. Die Aktivierung von T-Lymphozyten mit den beschriebenen Begleiterscheinungen ist Teil der physiologischen Immunreaktion, kann dort aber in pathologischen Situationen außer Kontrolle geraten (lymphoproliferative Erkrankungen), oder unzureichend sein (Immundefizienz).

Modulation der Proliferation von T-Zellen meint entweder die Verstärkung der Aktivierung (bei pathologisch unzureichender Aktivierung) oder Abschwächung bzw. Inhibierung der Aktivierung (bei pathologisch lymphoproliferativen Erkrankungen).

Mehrere Untergruppen der T-Zellen meint zumindest die Untergruppen CD4 und CD8 T-Zellen.

Ein analoges Peptid ist ein Peptid, dessen Aminosäuresequenz von jener des Peptids, zu welchem es analog ist, abweicht, jedoch einen definierten Bindungspartner mit zumindest gleicher Affinität bindet. Abweichungen in der Sequenz können Deletionen, Substitutionen, Insertionen und Elongationen sein. Ein analoges Peptid wird in der Regel eine dem Peptid sehr ähnliche Tertiär(teil)struktur und/oder Exposition im Rahmen eines (Zelloberflächen-) Proteins aufweisen und braucht ansonsten nur im dem dem unmittelbaren Bindungsbereich des Peptids analogen Bereich eine Bindungsstelle für den definierten Bindungspartner aufzuweisen bzw. diese bilden.

Eine Mimikriverbindung eines mAb ist eine natürliche oder synthetische chemische Struktur, die sich in einem Bindungsassay wie ein definierter mAb verhält, welchen die Mimikriverbindung mimikriert.

Eine Mimikriverbindung eines C'-D Loops ist eine natürliche oder synthetische chemische Struktur, an welche superagonistische und für ein Mitglied der CD28 Familie spezifische mAb spezifisch binden.

Der Begriff der mAb umfaßt neben Strukturen des üblichen Fab/Fc Aufbaus auch Strukturen, die ausschließlich aus dem Fab-Fragment bestehen. Es ist auch möglich, lediglich die variable Region zu Nutzen, wobei das Fragment der schweren Ketten mit dem Fragment der leichten Kette auf geeignete Weise, beispielsweise auch mittels synthetischer Brückenmoleküle, so verbunden ist, daß die Bindungsregionen der Ketten die Antikörperbindungsstelle bilden. Der Begriff der Antikörper umfaßt auch (vollständige) chimäre sowie humanisierte Antikörper.

Superagonistische Modulation der Proliferation von T-Zellen meint, daß keine Costimulation, i.e. kein weiteres Bindungsereignis neben einer Bindung eines mAb oder einer Mimikriverbindung an ein Mitglied der CD28 Familie zur Stimulation oder Inhibierung der Proliferation erforderlich ist.

Ein Screening Verfahren umfaßt dem Einsatz eines Targets, beispielsweise einer Teilsequenz aus CD28, wobei eine oder mehrere bekannte oder unbekannte Substanzen mit dem Target kontaktiert wird und ein Bindungsereignis festgestellt oder nicht festgestellt wird. Im Falle der Feststellung eines Bindungsereignisses wird die Substanz selektiert. Im Falle des Einsatzes einer Mischung von Substanzen schließt sich typischerweise einer Selektion der Mischung eine Dekonvolution an zwecks Ermittelung bindender Komponenten in der selektierten Mischung.

Als CD28 Familie ist eine Gruppe von T-Zelloberflächenrezeptoren bezeichnet, die immunregulatorische Aktivität aufweisen. Dies kann entweder stimulierend sein, wie im Falle des CD28 oder inhibierend, wie im Falle des CTLA-4. Zur CD28 Familie gehören CD28, CTLA-4, PD-1 und ICOS.

Ein Substrat kann löslich, unlöslich und/oder immobilisiert sein. Ein Substrat kann aus beliebigen natürlichen oder synthetischen Molekülen gebildet sein, u.a. aus Aminosäurenketten. Insofern muß ein Protein oder Peptid in der Terminologie dieses Textes nicht notwendigerweise ein Protein oder Peptid in üblicher Definition sein. In der Regel ist ein Protein oder Peptid nach der hier verwendeten Terminologie allerdings auch ein Protein oder Peptid in fachüblicher Terminologie.

Hintergrund der Erfindung und Stand der Technik.

Zum Verständnis der Erfindung ist zunächst folgender technologischer Hintergrund wichtig. Die Aktivierung ruhender T-Zellen zur Proliferation und funktionellen Differenzierung erfordert zunächst die Besetzung zweier Oberflächenstrukturen, sogenannter Rezeptoren: 1. des Antigenrezeptors, der von Zelle zu Zelle eine unterschiedliche Spezifität besitzt und für die Erkennung von Antigenen, z. B. viralen Spaltprodukten, notwendig ist; sowie des auf allen ruhenden T-Zellen mit Ausnahme einer Untergruppe der CD8 T-Zellen des Menschen gleichermaßen exprimierten CD28 Moleküls, welches natürlicherweise an Liganden auf der Oberfläche anderer Zellen des Immunsystems bindet. Man spricht von der Costimulation der antigenspezifischen Immunreaktion durch CD28. In Zellkultur können diese Vorgänge nachgestellt werden durch Besetzung des Antigenrezeptors sowie des CD28-Moleküls mit geeigneten mAbs. Im klassischen System der Costimulation führt weder die Besetzung des Antigenrezeptors noch die des CD28-Moleküls allein zur T-Zellproliferation, die Besetzung beider Rezeptoren ist jedoch effektiv. Diese Beobachtung wurde an T-Zellen des Menschen, der Maus und der Ratte gemacht.

Dagegen sind auch mAbs bekannt, die allein die T-Zellproliferation einleiten können. Eine solche superagonistische, d. h. von der Besetzung des Antigenrezeptors unabhängige Aktivierung ruhender T-Lymphozyten durch CD28-spezifische mAb, wurde in folgenden Systemen beobachtet: in der Literaturstelle Brinkmann et al., J. Immunology, 1996, 156: 4100-4106 wurde gezeigt, daß ein sehr kleiner Anteil (5 %) menschlicher T-Lymphozyten, die den für ruhende T-Lymphozyten typischen Oberflächenmarker CD45 RO tragen, durch den normalerweise Costimulation erfordernden CD28-spezifischen mAb 9.3 bei Zusatz des Wachstumsfaktors Interleukin-2 (IL-2) ohne Besetzung des Antigenrezeptors aktiviert wird. In der Arbeit von Siefken et al., Cellular Immunology, 1997, 176: 59-65, wurde gezeigt, daß ein auf konventionellem Wege, d.h. durch Immunisierung von Mäusen mit menschlichen T.-Zellen, hergestellter CD28-spezifischer mAb in Zellkultur eine Untergruppe menschlicher T-Zellen ohne Besetzung des Antigenrezeptors zur Proliferation aktivieren kann, wenn CD28 durch diesen mAb besetzt wird und die zellgebundenen mAb zusätzlich durch weitere Antikörper miteinander vernetzt werden. Den insofern bekannten Antikörpern ist gemeinsam, daß nur ein kleiner Anteil der T-Zellen aktivierbar ist.

In der Arbeit von Tacke et al., Eur. J. Immunol., 1997, 27:239-247 wurden zwei Arten von CD28-spezifischen mono-klonalen Antikörpern mit unterschiedlichen funktionellen Eigenschaften beschrieben: costimulatorische mAb, die die Aktivierung ruhender T-Zellen nur bei gleichzeitiger Besetzung des Antigenrezeptors costimulieren; und superagonistische mAb, die ohne Besetzung des Antigenrezeptors T-Lymphozyten aller Klassen in vitro und im Versuchstier zur Proliferation aktivieren können. Beide insofern bekannte mAb rühren aus einer Immunisierung mit Zellen, auf denen Ratten-CD28 exprimiert ist und sind durch auf ihre jeweiligen beschriebenen Eigenschaften gerichtete unterschiedlichen Selektionen erhältlich. Schließlich ist aus der Literaturstelle WO 98/54225 ein weiterer superagonistischer mAb bekannt, nämlich CMY-2.

Die insofern bekannten superagonistischen mAb genügen in ihrer stimulatorischen Wirkung jedoch noch nicht den Anforderungen hinsichtlich der Stärke des aktivierenden Effektes oder der Breite der aktivierten Subpopulationen von T-Lymphozyten oder weisen nicht die erforderliche Humanspezifität auf.

Proteine enthaltend Seq. -ID 41 und/oder Saq.-ID 1 sind aus dem Literaturstellen EP 0947582 C und WO-0171042 A bekannt.

### Technisches Problem der Erfindung.

Der Erfindung liegt daher das technische Problem zu Grunde Mittel anzugeben, mit welchen sich superagonistische Substanzen, die an eines oder mehrere Mitglieder der CD28 Familie spezifisch binden und eine verbesserte stimulatorische oder inhibierende Wirkung aufweisen, finden lassen, sowie solche Substanzen anzugeben.

Die der Erfindung zu Grunde liegende Erkenntnis.

Der Erfindung liegt die Untersuchung der Bindungsbereiche von superagonistischen mAb an CD28 zu Grunde sowie die im zuge dieser Untersuchungen gefundene Wechselwirkung des C'-DLoop von CD28 mit superagonistischen mAb. Der Erfindung liegt die weitere Erkenntnis zu Grunde, daß ein entsprechender Bindungsbereich für superagonistische mAb in den anderen Mitgliedern der CD28 Familie zu finden ist, nämlich auch dort die C'-D Loops. Aus dieser grundsätzlichen Erkenntnis leiten sich verschiedene Aspekte für technische Lehren der Erfindung her.

Grundzüge der Erfindung und bevorzugte Ausführungsformen.

Zur Lösung dieses technischen Problems lehrt die Erfindung dem gegenstand des Anspruchs 1. Mit anderen Worten ausgedrückt, ist das wesentliche Element eines erfindungsgemäßen Proteins oder Peptids die C'-D Loop Struktur (oder eines Analogen/einer Mimikri hierzu), und zwar unabhängig davon, ob und welche Sequenzen sich beidseitig des Loops anschließen. Wesentlich ist lediglich, daß die Loop Struktur hinreichend exponiert ist, um Zugang für superagonistische mAb oder Mimikriverbindungen zu bieten und im Falle der spezifischen Bindungsmöglichkeit eine Bindung nicht aus sterischen Gründen zu verhindern. Im Rahmen der Erfindung ist es dabei überraschend, daß alle gefundenen superagonistischen CD28 spezifischen mAb an den C'-D Loop binden, während nicht-superagonistische CD28 spezifische mAb nicht dorthin binden. Mit einer einzigen Gruppe von Zielstrukturen hinsichtlich ihrer räumlichen Anordnung auf Mitgliedern der CD28 Familie werden somit prospektive Wirkstoffe gegen die unterschiedlichsten Erkrankungen zugänglich.

In der Ausführungsform eines Peptids, insbesondere eines Oligopeptids (4 - 9 Aminosäuren) oder Polypeptids (10 - 100 Aminosäuren) ist es bevorzugt, wenn dessen Enden an jeweils eine Bindungsstelle eines Substrats gebunden sind, wobei die Bindungsstellen des Subtrats räumlich zueinander gemäß den Bindungsstellen für das C'-D Loop angeordnet sind, wobei das C'-D Loop oder das hierzu analoge Peptid in einer dreidimensionalen Konfiguration gemäß dem C'-D Loop fixiert ist, wobei das gebundene C'-D Loop oder das hierzu analoge Peptid für Antikörper frei zugänglich sind, und wobei das Substrat nicht ein Mitglied der CD28 Familie ohne C'-D Loop ist. Hierdurch wird eine eine Bindung mit superagonistischen Substanzen ermöglichende dreidimensionale Struktur eingerichtet.

Im Einzelnen kann ein erfindungsgemäß verwendetes peptid oder Protein eine Aminosäurensequenz Seq.-ID 41 (Human CD28 Loop), nicht jedoch Human CD28 , enthalten. Hieran können am 3'-Ende und/oder am 5'-Ende ein oder zwei Aminosäuren gemäß Fig. 7 angehängt sein. Aber auch Teilsequenzen daraus können in erfindungsgemäßen Peptiden enthalten sein, beispielsweise gemäß der Sequenz Seq.-ID 1. Die Seq.-ID 5 bis 10 geben Varianten des Human CD28 Loops an. An eine Sequenz 1 kann eine oder mehrere Aminosäuren der Sequenz 11 gemäß der Fig. 7a angeschlossen sein. Bei den genannten Sequenzen handelt es sich um erfindungsgemäße Bereiche, an welche superagonistische mAb spezifisch binden. Man erkennt bei Vergleich der Sequenzen insbesondere, daß die Primärstruktur des Loops für die jeweiligen Familienmitglieder spezifisch ist. Durch Auswahl des C'-D Loops eines bestimmten Mitgliedes und somit durch Einsatz von Substanzen mit Spezifität für dieses ausgewählte Loop kann somit gezielt alternativ eine Aktivierung oder Inhibierung der Proliferation bewirkt werden.

Ein (CD28 spezifisches) erfindungsgemäßes Protein oder Peptid hierzu läßt sich dadurch identifizieren, daß ein oder mehrere prospektive Proteine oder Peptide einem Bindungstest mit einem der mAb 9D7 oder 5.11A unterworfen und bindende Peptide selektiert werden. Bei den genannten mAb handelt es sich um neue superagonistische CD28 spezifische mAb, die im experimentellen Teil im Detail beschrieben werden. Mittels entsprechender mAb mit Spezifität gegenüber einem C'-D Loop eines anderen CD28 Familienmitgliedes lassen sich für die anderen Mitglieder spezifische erfindungsgemäße Proteine, Peptide oder Mimikri Verbindungen identifizieren.

Beschrieben wird weiterhin eine Nukleinsäure codierend für ein erfindungsgemäß verwendetes Peptid oder für ein ein solches Peptid enthaltendes Protein, nicht jedoch codierend für ein Mitglied der CD28 Familie, sowie ein plasmid enthaltend eine solche Nukleinsäure.

Ein erfindungsgemäßes Peptid oder Protein kann in einem Verfahren zur Herstellung von mAb eingesetzt werden, die superagonistisch die Proliferation von T-Zellen mehrerer bis aller Untergruppen modulieren, wobei ein nichtmenschliches Säugetier mit dem Peptid, Protein, oder der Mimkriverbindung hierzu immunisiert wird, wobei aus dem nichtmenschlichen Säugetier Zellen entnommen und aus den Zellen Hybridomzellen hergestellt werden, und wobei die so erhaltenen Hybridomzellen mit der Maßgabe selektiert werden, daß in deren Kulturüberstand mAb enthalten sind, die an das C'-D Loop des Peptids oder Proteins binden, solche Hybridomzellen sowie mAb erhältlich mit solchen Hybridomzellen. Erfindungsgemäße mAb aus dem Menschen lassen sich alternativ aber auch dadurch herstellen, daß B-Lymphozyten selektiert werden, welche an das Loop binden, und deren exprimierte Immunglobulingene kloniert werden. Weiterhin lassen erfindungsgemäße menschliche mAb aus Phagenbibliotheken isolieren. Der Durchschnittsfachmann ist mit seinen Kenntnissen ohne weiteres in der Lage, solche Verfahren auszuführen, so daß von einer detaillierten Beschreibung hier abgesehen werden kann.

Mit der Erfindung lassen sich neue superagonistische CD28 Familien-spezifische mAb identifizieren. Daher betrifft die Erfindung auch die Verwendung eines erfindungsgemäßen peptids, Proteins, oder einer erfindungsgemäßen Mimikriverbindung hierzu in einem Screeningverfahren zur Identifizierung von Substanzen, die superagonistisch die Proliferation von T-Zellen mehrerer bis aller Untergruppen modulieren, wobei eine prospektive Substanz oder eine Mischung von prospektiven Substanzen einem Bindungsassay mit dem Peptid oder Protein unterworfen werden, und wobei an das Peptid oder Protein bindende Substanzen selektiert werden. Eingesetzt werden können grundsätzlich alle fachüblichen Bindungsassays. Von besondere Bedeutung kann hierbei die Suche nach Mimikriverbindungen sein, da diese typischerweise sogenannte small molecules sind, die gegenüber Makromolekülen pharmakologische Vorteile aufweisen. In einem solchen Screening Verfahren nach Mimikri Verbindungen kann mit hohem Durchsatz eine Stoffbibliothek gescreent werden.

Sowohl ein erfindungsgemäßgefundenes Peptid, Protein oder Mimikri Verbindung hierzu als auch die erfindungsgemäßen mAb haben therapeutische Bedeutung, da damit lymphoproliferative Erkrankungen durch Inhibierung der Proliferation ebenso wie Immundefizienz-Erkrankungen durch Aktivierung der Proliferation behandelt werden können. Auch die Induktion von Effektorfunktionen, z.B. Sekretion von Effektorsubstanzen, ist möglich. Dies erfolgt durch Selektion oder Design des mAb oder der Mimikriverbindung nach Maßgabe einer Spezifität und hohen Affinität für ein bestimmtes Mitglied der CD28 Familie. Ist eine weiter erhöhte Spezifität/Affinität wünschenwert , beispielsweise zur Beherrschung überraschender Nebenwirkungen, so kann so vorgegangen werden, daß ein zweiter Ligand neben dem mAb oder der Mimikriverbindung mit Spezifität für das spezielle Familienmitglied gesucht wird, und der zweite Ligand nach Analyse der relativen räumlichen Positionen der beiden Liganden zueinander über eine Brückenverbindung mit dem mAb bzw. der Mimikriverbindung verknüpft wird und so beide Liganden gleichsam gegeneinander in Bindungsstellung ausgesteift werden. Die Bestimmung der räumlichen Position zweier Liganden zueinander nach Bindung an ein Target kann beispielsweise mit Röntgenstrukturanalyse oder mehrdimensionaler NMR Korrelationsspektroskopie, beispielsweise ¹⁵N/¹H NMR, erfolgen. Ein zweiter Ligand läßt sich mittels üblicher Screening Methoden ermitteln, wobei das spezielle CD28 Familienmitglied als Target eingesetzt wird. Es versteht sich, daß der zweite Ligand nicht am C'-D Loop bindet, sondern räumlich hiervon beabstandet. Auf der anderen Seite ist es möglich, daß ein erfindungsgemäßgefundenes Peptid, Protein oder eine Mimikriverbindung hierzu, welches keine sonstige physiologische Wirkung aufweist, natürliche Liganden der Mitglieder der CD28 Familie kompetitiv bindet und so durch Verhinderung einer pathologisch bedingten natürlichen Aktivierung oder Inhibierung einen reversen Effekt erzielt.

Andererseits betrifft daher die Erfindung die Verwendung eines erfindungsgemäßen mAb zur Herstellung einer pharmazeutischen Zusammensetzung zur Behandlung von Erkrankungen mit pathologisch erniedrigten CD4-T-Zellzahlen, insbesondere AIDS, zur Herstellung einer pharmazeutischen Zusammensetzung zur Behandlung nach Stammzelltransplantationen in Verfolg von Chemo- oder Strahlentherapie bei leukämischen Erkrankungen, zur Herstellung einer pharmazeutischen Zusammensetzung zur Potenzierung und/oder qualitativen Beeinflussung von Immunreaktionen bei Schutzimpfungen, und zur Herstellung einer pharmazeutischen Zusammensetzung zur Behandlung von autoimmun-inflammatorischen Erkrankungen.

Im Folgenden wird die Erfindung anhand von lediglich Ausführungsformen darstellenden Beispielen näher erläutert. Es zeigen:
- Fig. 1:: Stimulation von T-Lymphozyten der Ratte mit verschiedenen CD28 spezifischen mAb (a: Costimulation, b: superagonistische, i.e. direkte, Stimulation),
- Fig. 2:: einen Sequenzvergleich zwischen Maus, Ratte und Human CD28 im Bereich des C'-D Loops (eingekastelt),
- Fig. 3:: experimentelle Ergebnisse zur Lokalisierung der Bindungsstelle superagonistischer mAb am CD28 Moleküle der Ratte,
- Fig. 4:: Bindung von verschiedenen human CD28 spezifischen mAb an CD28 (a) sowie costimulatorische (b) und superagonistische, i.e. direkt stimulatorische (c) Aktivität der mAb aus Figur 4a,
- Fig. 5:: Bindungsversuche, die belegen, daß human CD28 spezifische superagonistische mAb spezifisch an das C'-D Loop binden,
- Fig. 6:: eine dreidimensionale Darstellung von CD28 mit Markierung des C'-D Loop,
- Fig. 7:: Sequenzalignment unter Hervorhebung des C'-D Loop bzw. analoger Strukturen, für verschiedene Mitglieder der CD28 Familie, und
- Fig. 8:: Experimente zur Aktivierung von Zellen mittels erfindungsgemäßer human CD28 spezifischer mAb und mutierter Maus CD28 Moleküle.
- Fig. 9:: Darstellung der Sequenzen SEQ-ID 33 - 40 (a - h)
- Fig. 10:: Humanisierte variable Domäne des mAb 5.11A (leichte Kette: VLC5.11=a; schwere Kette: VHC5.11=b)

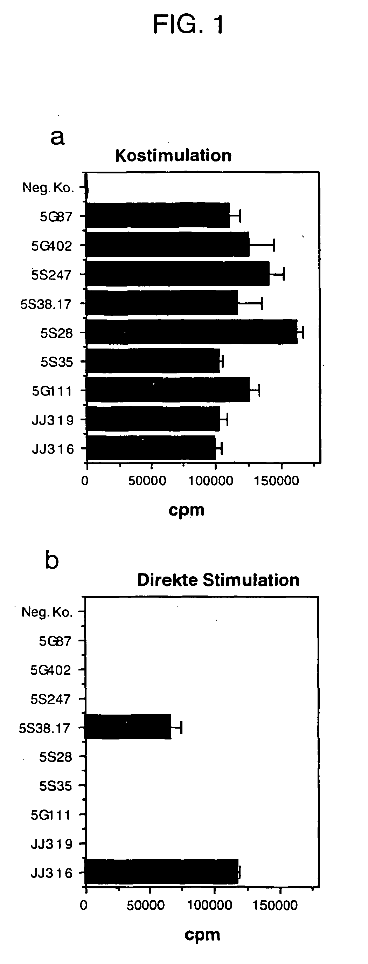

Fig. 1 zeigt die Stimulation frisch isolierter T-Lymphozyten der Ratte in Form eines 3H-Thymidin-Einbaus. Die Methodik entspricht jener, wie in der Literaturstelle WO98/54225 beschrieben, auf welche hier und folgend vollumfänglich Bezug genommen wird und deren Offenbarungsinhalt hiermit in den vorliegenden Text inkorporiert wird. In der Fig. 1a ist die Costimulation gezeigt, d. h. in allen Näpfen waren T-Zellrezeptor (TCR)-spezifische mAb an die Plastikoberfläche gebunden. Mangels Costimulation zeigt die Negativkontrolle (oberste Reihe) keinen Einbau. Costimulation wird sodann durch die Zugabe CD28-spezifischer mAb in löslicher Form gegeben. Zum Einsatz kam die gesamte gezeigte Palette CD28-spezifischer mAb. Diese Serie von verschiedenen CD28-spezifischen mAb stammt aus einem in WO98/54225 bereits beschriebenen Ansatz der Immunisierung und Herstellung von Hybridom-Zelllinien. Es handelt sich um Kulturüberstände, die genug CD28-spezifische mAb für eine sättigende Bindung an die pro kultivierten 2×10^5 T-Zellen enthielten. Der Figur 1a ist entnehmbar, daß sämtliche dieser mAb in der Lage sind, costimulierend zu aktivieren, d.h. in Anwesenheit der anti-TCR mAb den Thymidin-Einbau anzuregen. In Fig. 1b ist die Stimulation in Abwesenheit TCR-spezifischer mAb gezeigt. Auch dieses Experiment wurde so durchgeführt, wie in der Literaturstelle WO98/54225 beschrieben. Man erkennt, daß nur zwei mAb in der Lage sind, in Abwesenheit eines TCR-Signals die T-Lymphozyten zu stimulieren. Diese mAb besitzen also superagonistische Aktivität.

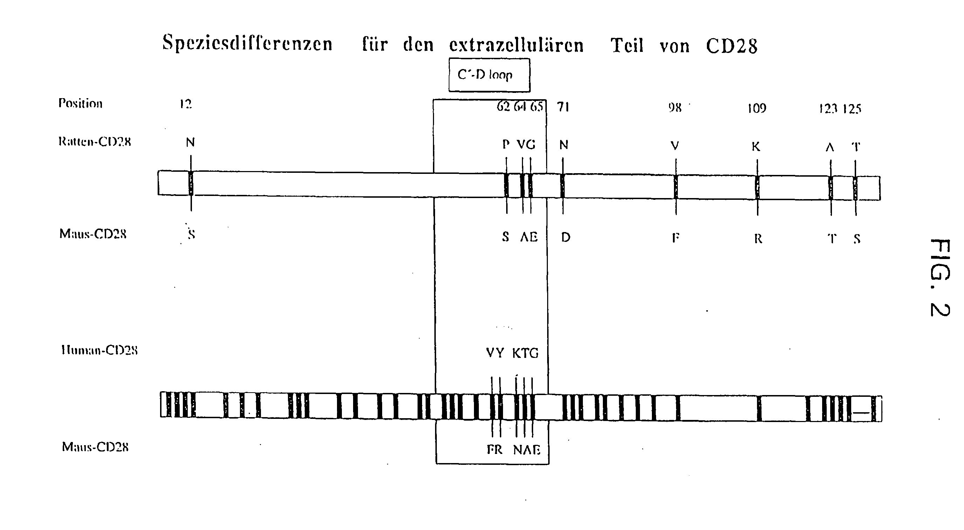

Im Weiteren wurde untersucht, ob costimulatorische und superagonistische CD28-spezifische mAb an unterschiedliche Bereiche des CD28-Moleküls binden. Die mAb wurden durch Immunisierung von Mäusen mit CD28 der Ratte hergestellt; erwartungsgemäß reagieren sie deshalb alle nicht mit Maus CD28 (nicht gezeigt). Da die mAb also nur solche Bereiche des Ratten CD-28-Moleküls erkennen können, die sich von dem der Maus unterscheiden, wurde zunächst ein Sequenzvergleich zwischen CD28 der Maus und der Ratte vorgenommen (siehe Fig. 2, oberer Teil). Die Unterschiede zwischen beiden Spezies sind hervorgehoben. Zur Benennung der Aminosäuren wurde der Ein-Buchstaben-Code verwendet. Als Prototypen für einen konventionellen Ratten-CD28-spezifischen mAb wurde JJ319, für einen superagonistischen mAb wurde JJ316 verwendet (siehe WO98/59225).

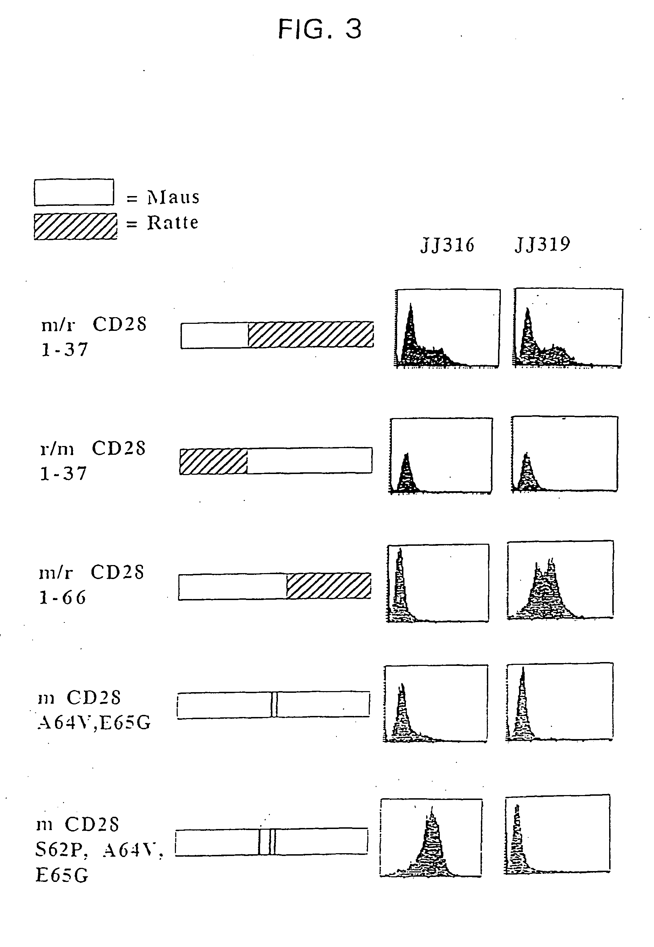

In Figur 3 ist die Kartierung der Bindung gezeigt. Es wurden Expressionsplasmide konstruiert, in denen ein Teil der extrazellulären Domäne von CD28 aus der Maus, ein anderer aus der Ratte stammt. Dies ist jeweils symbolisch durch Balken bzw. Striche dargestellt; rechts daneben ist die Bindung der mAb JJ316 und JJ319 an Maus-Fibroblasten (L929-Zellen) gezeigt, die mit diesen Expressionsplasmiden transfiziert worden waren. In den ersten beiden Zeilen der Abb. 3 (m/r und r/m 1-37) wird die Bindung beider Antikörper zur "rechten" Hälfte der Sequenz kartiert: Beide binden, wenn diese aus der Ratte stammt; Im umgekehrten Konstrukt (rm CD28 1-37, links Ratte, rechts Maus) findet keine Bindung statt. In der dritten Zeile (m/r CD28 1-66) wird gezeigt, dass JJ316 nicht mehr bindet, während der noch vorhandene Teil der Rattensequenz ("rechts") für die Erkennung durch JJ319 noch ausreicht. Demnach erkennen die beiden mAb unterschiedliche Epitope auf dem CD28-Molekül, und die Bindung des Superagonisten JJ316 muss folglich in dem Bereich zu suchen sein, der in dem Konstrukt der ersten Zeile, nicht aber in dem Konstrukt der dritten Zeile aus der Ratte stammte. Klarer Kandidat dafür ist der in Figur 2 eingekastelte Bereich.

In den Zeilen 4 und 5 der Figur 3 wurden deshalb zunächst zwei und dann drei Aminosäuren in diesem Bereich des Maus CD28-Moleküls so verändert, dass sie nun die Rattensequenz darstellen. Durch diese "Transplantation" von nur drei Aminosäuren konnte die Bindungsfähigkeit für mAb JJ316, nicht aber (wie erwartet) die von JJ319 übertragen werden. In Tab. 1 sind die Bindungsdaten für die ganze Palette CD28-spezifischer mAb zusammengefasst. Es ergibt sich eine eindeutige Korrelation: eine signifikante Bindung an den C'-D Loop der Ratte, der durch Übertragung der Aminosäurenpositionen 62, 64 und 65 auf das CD28 Molekül der Maus geschaffen wurde, zeigte sich nur für die beiden superagonistischen mAb JJ316 und 5S38.17, für die konventionellen (nur costimulatorischen) mAb dagegen nicht. Ein costimulatorischer mAb (5S35) erkennt das eingekastelte Epitop sehr schwach und bindet sehr stark an das "konventionelle" Epitop.

Die nächsten Figuren beschäftigen sich mit superagonistischen human-spezifischen mAb. Auch diese wurden in Mäusen hergestellt, reagieren also nicht mit dem CD28-Molekül der Maus. Die Mäuse wurden mit human-CD28-transfizierten A20/J Maus B-Lymphomzellen immunisiert (siehe WO98/54225) und zusätzlich vor der Fusion mit käuflich erhältlichem human CD28-Fc Fusionsprotein geboostert (von R und D Systems gekauft). In einer Serie von Fusionsexperimenten wurden aus mehreren Tausend Zelllinien 24 identifiziert, die human-CD28 spezifische mAb produzieren (Bindung an Maus L929 Zellen, die human-CD28 exprimieren, aber nicht an untransfizierte L929 Zellen), analog zum Screen in der Literaturstelle WO98/54225. Von diesen zeigten zwei die gesuchte superagonistische Aktivität (9D7 und 5.11A), während alle neuen mAb konventionelle costimulatorische Aktivität besitzen. Im Weiteren werden insbesondere die beiden superagonistischen mAb beschrieben. Als Beispiel für einen konventionelle human-CD28-spezifischen mAb wird der ebenfalls neu generierte mAb 7.3B6 verwendet.

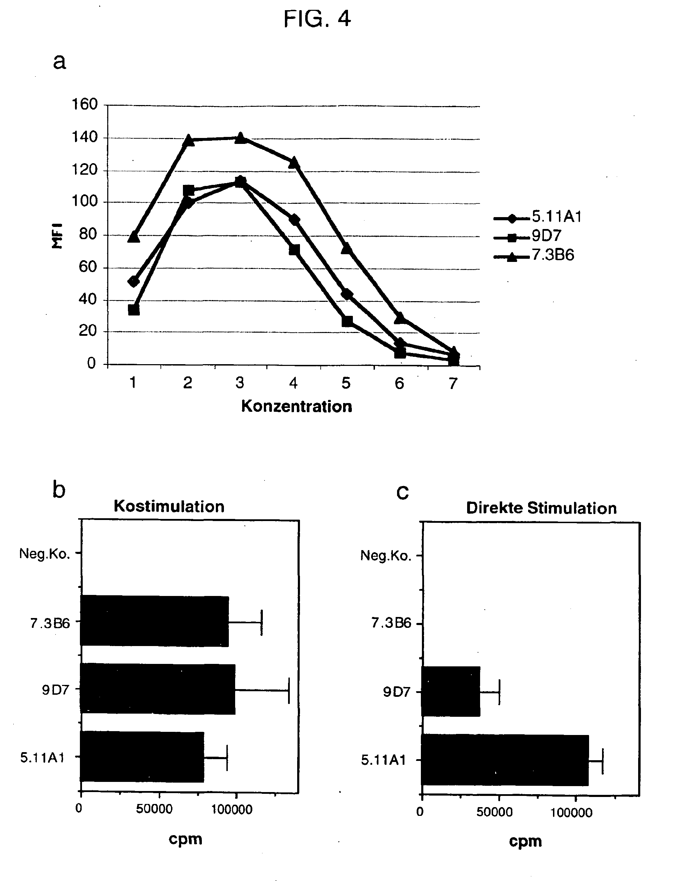

Figur 4a zeigt, dass die verwendeten Präparate der drei neuen mAb vergleichbar gut und auch mit vergleichbarem Titer an menschliche T-Lymphozyten binden. Gezeigt ist ein Experiment, in dem frisch isolierte mononukleäre Zellen aus dem menschlichen Blut (sog. PBMC) zunächst mit verschiedenen Verdünnungsstufen der eingesetzten mAb auf Eis behandelt wurden; dann wurde gewaschen und der gebundene mAb durch einen Fluoreszenz-Farbstoff markierten Sekundärantikörper sichtbar gemacht, der spezifisch die gebundenen Maus mAb erkennt. Durch Verwendung eines weiteren mAb, der menschliche CD4 T-Zellen detektiert und an den ein zweiter Fluoreszenz-Farbstoff gebunden war, konnte die Bindung der titrierten mAb durch elektronisches gating selektiv für CD4 T-Lymphozyten bestimmt werden. Angegeben ist mit "MFI" die durchschnittliche Fluoreszenzintensität, die ein Maß für die Menge des gebundenen CD28-spezifischen mAb darstellt. Die Konzentrationen stellen 3-fach Verdünnungen eines standardisierten Ausgangspräparats dar. Es ist durchaus normal, dass bei diesem Test die höchste Konzentration ein schwächeres Signal gibt als die folgenden Titrationsschritte; dies hat mit der Avidität (bivalente Bindung) von mAb zu tun und spielt in den hier diskutierten Zusammenhängen keinerlei Rolle.

In Figur 4b und c ist die Fähigkeit dieser drei neuen CD28-spezifischen mAb verglichen, - in An- und Abwesenheit eines TCR-Signals - frisch isolierte menschliche T-Zellen zum Wachstum zu stimulieren. Gezeigt ist wiederum ein 3H-Thymidin Einbau, wie vorstehend für die Ratte beschrieben. Für Figur 4b waren die Näpfe mit einem mAb beschichtet, der mit dem menschlichen TCR/CD3-Komplex reagiert. Es wurde also Costimulation gemessen. Man erkennt, daß die Proliferation ohne Costimulation mit einem der mAb ausbleibt (Negativkontrolle), alle drei Antikörper sind jedoch in der Lage, die Zellteilung zu stimulieren. Für Figur 4c wurde in Abwesenheit eines TCR/CD3-spezifischen mAb gearbeitet. Nur die Antikörper 9D7 und 5.11A konnten superagonistisch stimulieren.

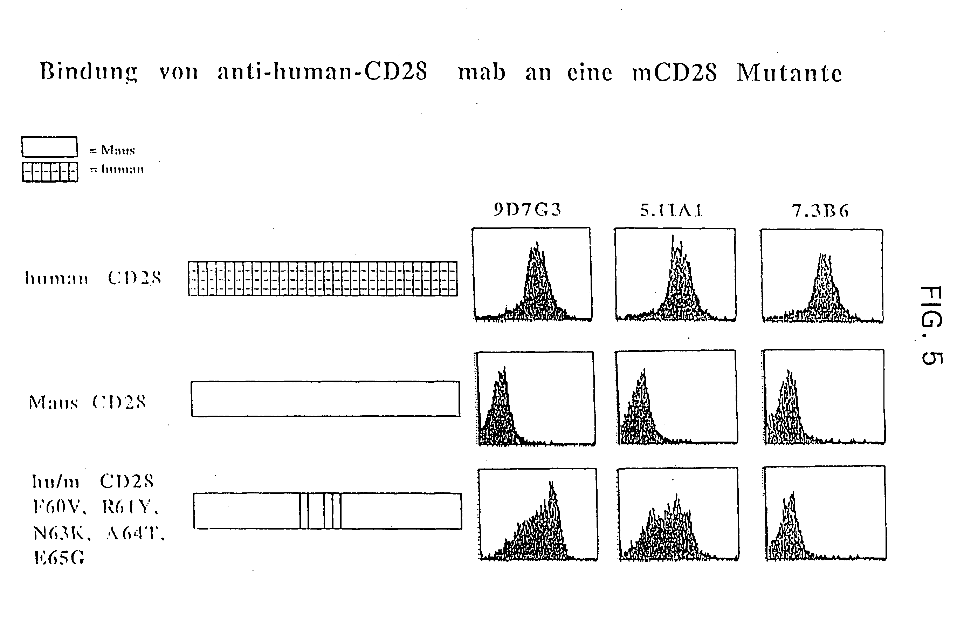

Nachdem das Epitop für superagonistische mAb bei der Ratte definiert ist und zwei neue superagonistische mAb mit Spezifität für menschliches CD28 isoliert worden waren, wurde überprüft, ob diese mAb an die entsprechende Stelle des menschlichen CD28-Moleküls binden. Wie aus Figur 2 ersichtlich, unterscheiden sich die CD28-Moleküle der Maus und des Menschen in zahlreichen Positionen. Aufbauend auf der Kartierung des superagonistischen Epitops bei der Ratte wurde deshalb direkt geprüft, ob sich die Bindungsstelle für das superagonistische Epitop auf menschlichem CD28 auf das CD28-Molekül der Maus durch "Transplantation" der fünf Aminosäuren dieses homologen Bereiches erreichen lässt. Die Ergebnisse sind in der Figur 5 gezeigt. Vor dem Hintergrund der homogen dargestellten Maus-Sequenz für die extrazelluläre Domäne des CD28 Moleküls (Mitte) sind als Striche die ausgetauschten (Maus zu human) Aminosäurepositionen dargestellt (unten). Die Zahlen an der Seite geben zusätzlich noch die einzelnen Positionen und Mutationen an (F60V bedeutet z.B., dass an Position 60 das Phenylalanin der Maus durch ein Valin der menschlichen Sequenz ersetzt wurde). Daneben ist die Bindung der drei untersuchten mAb dargestellt. Wie die Abbildung zeigt, erkennen zwar alle drei mAb menschliches CD28, nur die beiden mAb 9D7 und 5.11A reagieren jedoch mit dem Maus CD28-Molekül, dem die fünf Aminosäuren des menschlichen CD28 an der entscheidenden Stelle transplantiert worden waren. Angesichts der Vielfalt von Unterschieden ist diese gezielte Herstellung der Reaktivität überraschend und bestätigt in vollem Maße die aus den Experimenten mit Ratten CD28 abgeleitete Erkenntnis, dass superagonistische mAb an eine bestimmte, nämlich diese Stelle des Moleküls binden müssen.

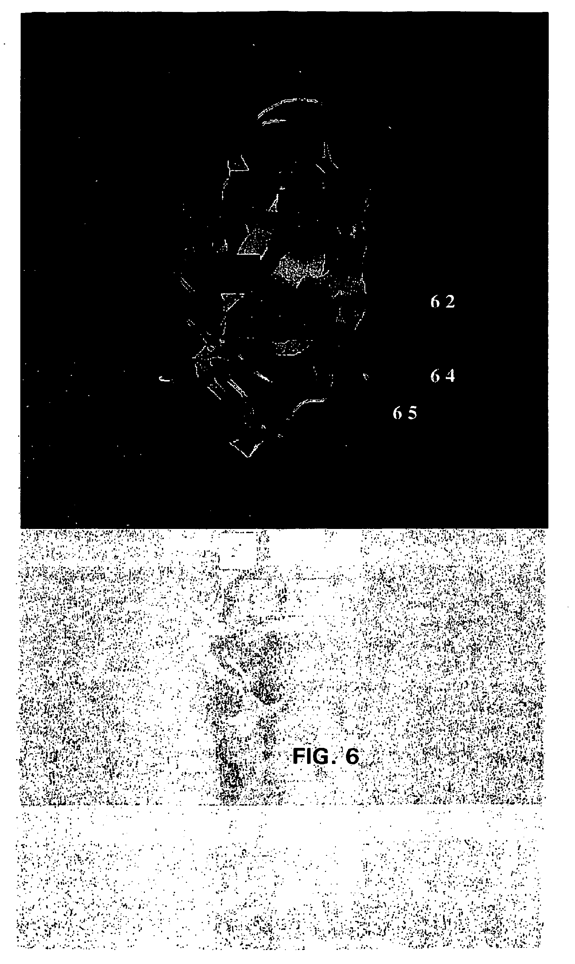

In Figur 6 ist ein dreidimensionales Modell des CD28-Moleküls gezeigt. Die neu identifizierte Bindungsregion ist hervorgehoben. Sie entspricht der eingekastelten Sequenz in Figur 2. Die extrazelluläre Domäne von CD28 gehört strukturell zur sog. Immunglobulin-Superfamilie, die sich durch zwei übereinanderliegende ß-Faltblätter als Grundstruktur auszeichnet. Die Beschriftung dieser Bänder erfolgt nach einem in der Literatur vorgegebenen Muster. Wichtig für die hier gezeigte Darstellung ist, dass die als Epitop für superagonistische CD28-spezifische mAb in Ratte und Maus identifizierte Region als "C'-D loop" bezeichnet wird. Es wurde also gezeigt, dass mAb mit Spezifität für den C'-D Loop des CD28-Moleküls superagonistische Aktivität besitzen, also im Sinne der Literaturstelle WO98/54225 zur Aktivierung von T-Lymphozyten eingesetzt werden können. Die superagonistische Aktivität C'-D Loop spezifischer mAb in Ratte und Mensch zeigt, dass es dabei nicht auf die Sequenz des Epitops, sondern auf seine Lage bzw.Form ankommt.

CD28 gehört zu einer Familie von Zelloberflächenrezeptoren mit immunregulatorischer Aktivität. Diese ist entweder stimulierend (CD28, ICOS), oder eine inhibierend (CTLA-4, PD-1). In Figur 7 sind die Sequenzen der bekannten Mitglieder der CD28-Familie im Sinne eines "alignment" zusammengestellt. Der C'-D Loop für CD28 ist hervorgehoben. Analoge Loops der anderen Moleküle (eingekastelt) sind eine entsprechend gute Zielstruktur für die Entwicklung superagonistischer Liganden. Zur Figur 7 ist anzumerken, daß "-" eine Lücke im Alignment darstellt, i.e. die daran anschließenden Aminosäuren unmittelbar miteinander verbunden sind.

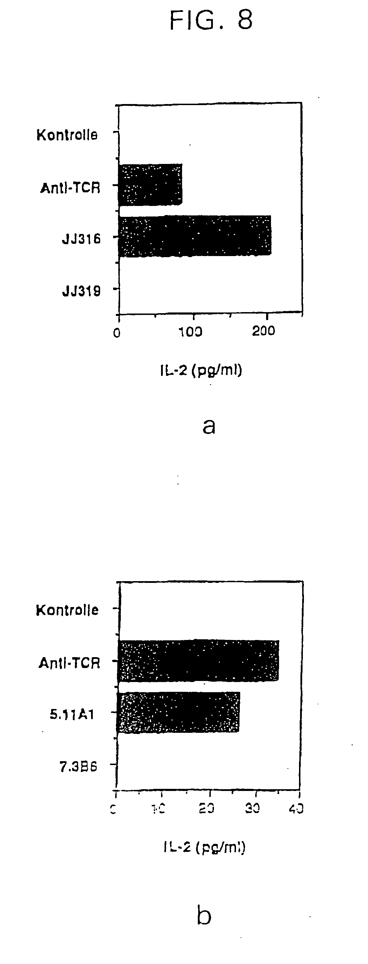

In den Experimenten der Fig. 8 wurde untersucht, ob erfindungsgemäße mAb mit Spezifität für den C'-D Loop der Ratte bzw. des Menschen nicht nur an die Maus CD28 mit "transplantiertem" C'-D Loop der Ratte bzw. des Menschen binden (siehe Figuren 3 und 5), sondern ob auch tatsächlich eine Aktivierung erfolgt. Hierfür wurden T-Tumorzellen der Maus, BW, entweder mit dem Konstrukt der Figur 3, Zeile 5, (Ratten C'-D Loop Übertragung) oder mit dem Konstrukt der Figur 5, Zeile 3, (human C'-D Loop) transfiziert. Die Aktivierung dieser Zellen wird nicht durch Zellteilung gemessen (sie proliferieren ohnehin), sondern durch die Produktion des Zytokins IL-2. Figur 8 zeigt zunächst, daß ohne Stimulation keine IL-2 Produktion erfolgt (Negativkontrolle). Stimulation mit einem T-Zellrezeptor-spezifischen mAb induziert IL-2 Produktion (Positivkontrolle). Figur 8a zeigt zeigt die Ergebnisse bei Einsatz des superagonistischen mAb JJ316 der Ratte, während Figur 8b Ergebnisse für den human C'-D Loop spezifischen mAb 5.11A zeigt. In beiden Fällen werden die jeweiligen Zelllinien zur IL-2 Produktion stimuliert. Erwartungsgemäß erfolgt die Stimulierung jedoch nicht mittels "konventioneller" CD28 spezifischer mAb, da diese nicht nur nicht an das C'-D Loop bindet, sondern das Kontrukt überhaupt nicht erkennen können, weil sie für ratten- bzw. humanspezifische Sequenzen spezifisch sind, die in dem Konstrukt nicht enthalten sind.

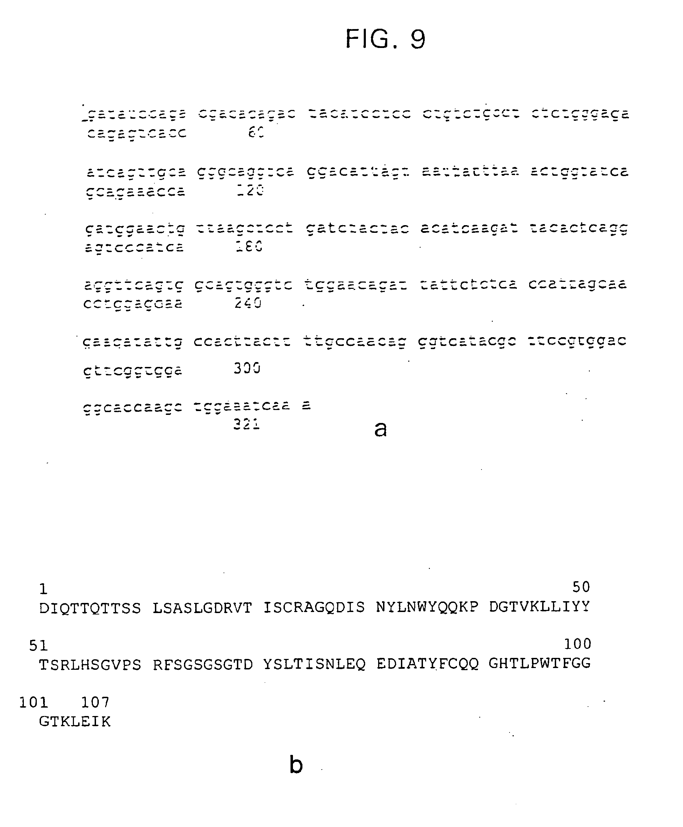

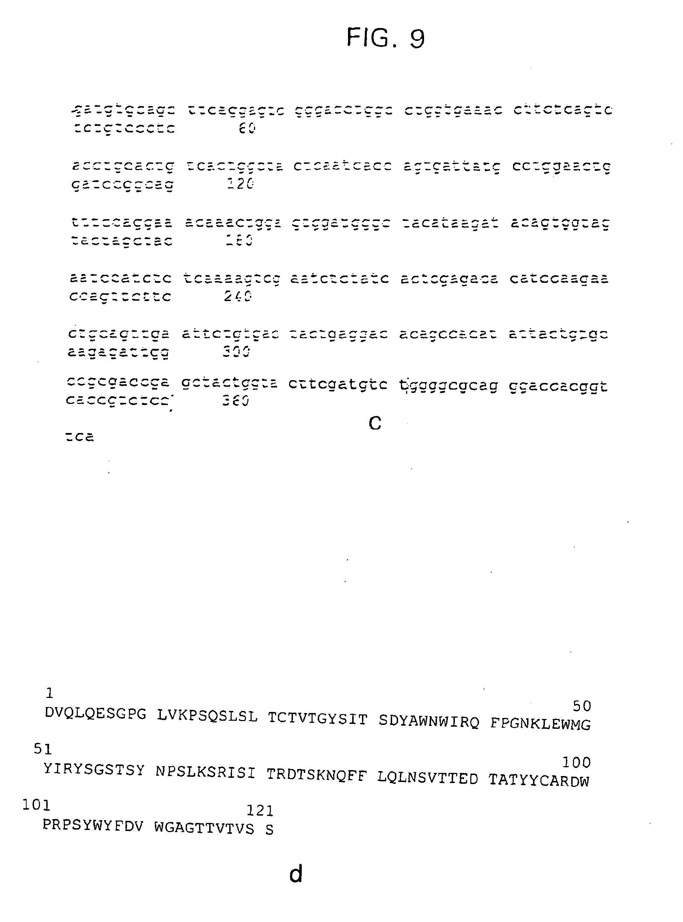

In der Figur 9a ist die Nukleinsäuresequenz der variablen Region der light chain eines erfindungsgemäßen mAb 9D7 wiedergegeben (SEQ-ID 33). Figur 9b zeigt das hierdurch codierte Peptid (SEQ-ID 34). Figur 9c zeigt die Nukleinsäuresequenz der variable Region der heavy chain dieses mAb (SEQ-ID 35). Figur 9d ist das hierdurch codierte Peptid (SEQ-ID 36).

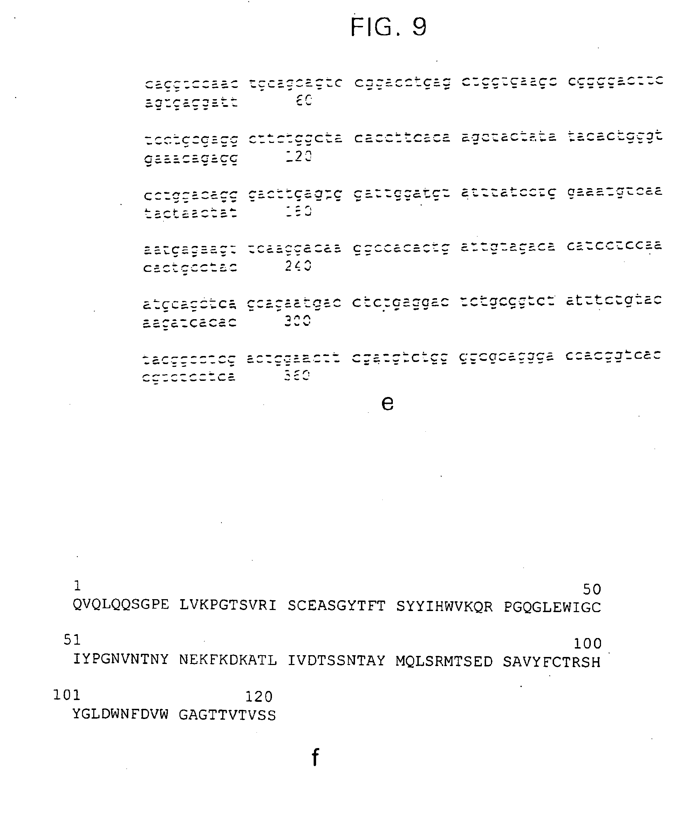

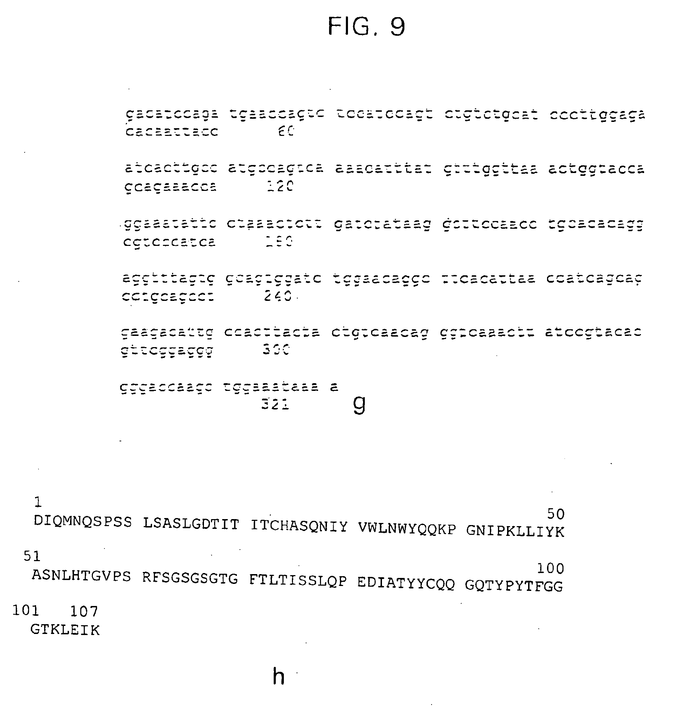

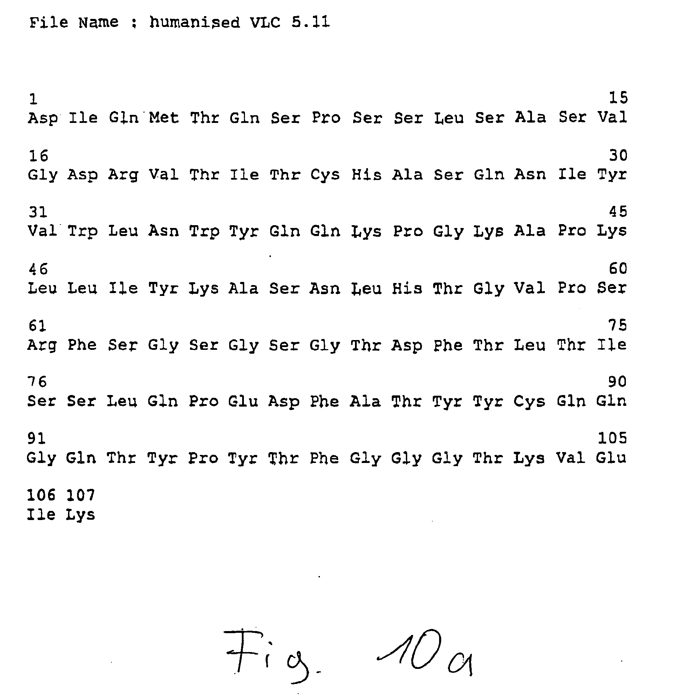

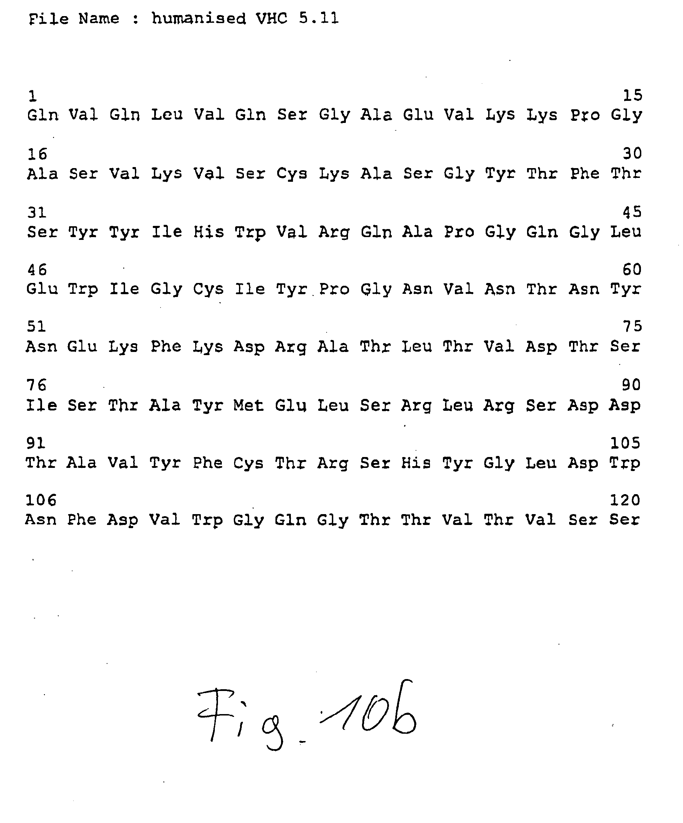

In der Figur 9e ist die Nukleinsäuresequenz der variablen Region der heavy chain eines erfindungsgemäßen mAb 5.11A wiedergegeben (SEQ-ID 37). Figur 9f zeigt das hierdurch codierte Peptid (SEQ-ID 38). Figur 9g zeigt die Nukleinsäuresequenz der variable Region der light chain dieses mAb (SEQ-ID 39). Figur 9h ist das hierdurch codierte Peptid (SEQ-ID 40). Figur 10 zeigt die humanisierte variable Domäne des mAb 5.11A. in Fig. 10a ist die leichte Kette und in Fig. 10b die schwere Kete dargestellt.

**Tabelle I:**

| Bindung von anti-Ratten CD28 mAb an Maus- und Ratten CD28 und verschiedene CD28 Mutanten. | | | | |
|---|---|---|---|---|
| mAb | Maus CD28 | Ratten CD28 | mCD28, S62P A64V, E65G | m/rCD28 Mva1269I |
| Kontr. | - | | - | - |
| JJ316 | - | +++ | +++ | - |
| JJ319 | - | +++ | - | +++ |
| 5S28 | - | ++ | - | ++ |
| 5S38.17 | - | +++ | +++ | - |
| 5S247 | - | +++ | - | +++ |
| 5G40/3 | - | +++ | - | +++ |
| 5G87 | - | ++ | - | ++ |
| 5G111 | - | ++ | - | ++ |
| 5S35 | - | +++ | + | +++ |

## Patentansprüche

1. Verwendung eines Peptids enthaltend eine Aminosäurensequenz des C'-D Loops eines Mitgliedes der CD28 Familie, nämlich Seq.-ID 41, Seq.-ID 1, oder 5 - 10 oder eines dieses Peptid enthaltenden Proteins, nicht jedoch Human CD28, in einem Screeningverfahren zur Identifizierung von Substanzen, die superagonistisch die Proliferation von T-Zellen mehrerer bis aller Untergruppen modulieren,
wobei eine Substanz oder eine Mischung von Substanzen einem Bindungsassay mit dem Peptid oder Protein hierzu unterworfen werden,
und wobei an das Peptid oder Protein hierzu bindende Substanzen selektiert werden.

2. Verwendung nach Anspruch 1, wobei die Substanz ein monoklonaler Antikörper ist.

3. Hybridomzellen, welche an ein Peptid oder Protein gemäß Aminosäurensequenz Seq.-ID 41, Seq.-ID 1, oder 5 - 10 bindende monoklonale Antikörper produzieren.

4. Hybridomzellen nach Anspruch 3, wie wie hinterlegt unter den DSM Nummern DSM ACC2531 oder DSM ACC2530.

5. Monoklonaler Antikörper, erhältlich aus den Hybridomzellen nach Anspruch 3 oder 4, welche zumindest teilweise codiert werden durch eine oder mehrere der Sequenzen Seq.-ID 33, 35, 37 und/oder 39, oder monoklonaler Antikörper enthaltend oder bestehend aus eine oder mehrere Sequenzen Seq.-ID 34, 36, 38 und/oder 40.

6. Verwendung eines monoklonalen Antikörpers nach Anspruch 5 zur Herstellung einer pharmazeutischen Zusammensetzung zur Behandlung von AIDS, oder zur Behandlung nach Stammzelltransplantationen in Verfolg von Chemo- oder Strahlentherapie bei leukämischen Erkrankungen, oder zur Potenzierung und/oder qualitativen Beeinflussung von Immunreaktionen bei Schutzimpfungen oder zur Behandlung von autoimmun-inflammatorischen Erkrankungen.

## Claims

1. A use of a peptide containing an amino acid sequence of the C'-D loop of a member of the CD28 family, namely seq.-ID 41, seq.-ID 1, or 5 - 10 or of a protein containing this peptide, not however human CD28, in a screening method for identifying substances which superagonistically modulate the proliferation of T cells of several to all subgroups,
wherein a substance or a mixture of substances are subjected to a binding test with the peptide or the protein, and
wherein substances binding to the peptide or protein are selected.

2. The use according to claim 1, wherein the substance is a monoclonal antibody.

3. Hybridoma cells producing monoclonal antibodies binding to a peptide or protein according to amino acid sequence seq.-ID 41, seq.-ID 1 or 5 - 10.

4. Hybridoma cells according to claim 3, as filed under the DSM numbers DSM ACC2531 or DSM ACC2530.

5. A monoclonal antibody, obtainable from the hybridoma cells according to claim 3 or 4, which are coded at least partially by one or more of the sequences seq.-ID 33, 35, 37 and/or 39, or a monoclonal antibody, which contains or consists of one or more sequences seq.-ID 34, 36, 38 and/or 40.

6. The use of a monoclonal antibody according to claim 5 for producing a pharmaceutical composition for the treatment of AIDS, or for the treatment following stem cell transplantations after chemo or radio therapy of leukemic diseases, or for multiplying and/or qualitatively influencing immune reactions after vaccinations, or for the treatment of autoimmune-inflammatory diseases.

## Revendications

1. Utilisation d'un peptide contenant une séquence des acides aminés de la boucle C'-D d'un membre de la famille CD28, c'est-à-dire Séq.-ID 41, Séq.-ID 1, ou 5 - 10 ou d'une protéine contenant ce peptide, pas cependant CD28 humain, dans un procédé de criblage pour l'identification de substances, qui modulent de manière superagonistique la prolifération de cellules T de plusieurs à tous les sous-groupes,
dans laquelle une substance ou un mélange de substances sont soumises à un essai de liaison avec le peptide ou la protéine, et
dans laquelle des substances se liant au peptide ou à la protéine sont sélectionnées.

2. Utilisation selon la revendication 1, dans laquelle la substance est un anticorps monoclonal.

3. Cellules d'hybridome, qui produisent des anticorps monoclonaux se liant à un peptide ou une protéine selon la séquence d'acides aminés Séq.-ID 41, Séq.-ID 1 ou 5 - 10.

4. Cellules d'hybridome selon la revendication 3, comme déposées sous les numéros DSM ACC2531 ou DSM ACC2530.

5. Anticorps monoclonal qui peut être obtenu de cellules d'hybridome selon la revendication 3 ou 4, qui sont au moins partiellement codées par une ou plusieurs des séquences Séq.-ID 33, 35, 37 et/ou 39, ou anticorps monoclonal contenant ou consistant en une ou plusieurs des séquences Séq.-ID 34, 36, 38 et/ou 40.

6. Utilisation d'un anticorps monoclonal selon la revendication 5 pour la production d'une composition pharmaceutique pour le traitement du sida, ou pour le traitement après des transplantations de cellules souches après la chimio- ou radiothérapie en cas de maladies leucémiques, ou pour la multiplication et/ou l'influence qualitative de réponses biologiques en cas de vaccinations préventives ou pour le traitement de maladies auto-immunes et inflammatoires.


### SEQUENCE LISTING

<110> TeGenero GmbH
<120> Peptid oder Protein enthaltend ein C'-D Loop der CD28 Re
   zeptorfamilie
<130> UNW/DE/0107
<160> 44
<170> PatentIn version 3.1
<210> 1
   <211> 6
   <212> PRT
   <213> Artificial
<400> 1
   Val Tyr Ser Lys Thr Gly
   1               5
<210> 2
   <211> 5
   <212> PRT
   <213> Artificial
<400> 2
   Phe Leu Asp Asp Ser
   1               5
<210> 3
   <211> 6
   <212> PRT
   <213> Artificial
<40C> 3
<21cm> 4
   <211> 6
   <212> PRT
   <213> Artificial
<400> 4
<210> 5
   <211> 4
   <212> PRT
   <213> Artificial
<400> 5
   Tyr Ser Lys Thr
   1
<210> 6
   <211> 5
   <212> PRT
   <213> Artificial
<400> 6
   Tyr Ser Lys Thr Gly
   1               5
<210> 7
   <211> 5
   <212> PRT
   <213> Artificial
<400> 7
   Val Tyr Ser Lys Thr
   1               5
<210> 8
   <211> 6
   <212> PRT
   <213> Artificial
<400> 8
   Tyr Ser Lys Thr Gly Phe
   1 5
<210> 9
   <211> 7
   <212> PRT
   <213> Artificial
<400> 9
   Val Tyr Ser Lys Thr Gly Phe
   1 5
<210> 10
   <211> 5
   <212> PRT
   <213> Artificial
<400> 10
   Ser Lys Thr Gly Phe
   1 5
<210> 11
   <211> 8
   <212> PRT
   <213> Artificial
<400> 11
   Gly Asn Tyr Ser Gln Gln Leu Gln
   1 5
<210> 12
   <211> 4
   <212> PRT
   <213> Artificial
<400> 12
   Leu Asp Asp Ser
   1
<210> 13
   <211> 5
   <212> PRT
   <213> Artificial
<400> 13
   Leu Asp Asp Ser Ile
   1 5
<210> 14
   <211> 5
   <212> PRT
   <213> Artificial
<400> 14
   Phe Leu Asp Asp Ser
   1 5
<210> 15
   <211> 6
   <212> PRT
   <213> Artificial
<400> 15
   Leu Asp Asp Ser Ile Cys
   1 5
<210> 16
   <211> 7
   <212> PRT
   <213> Artificial
<400> 16
   Phe Leu Asp Asp Ser Ile Cys
   1 5
<210> 17
   <211> 5
   <212> PRT
   <213> Artificial
<400> 17
   Asp Asp Ser Ile Cys
   1 5
<210> 18
   <211> 8
   <212> PRT
   <213> Artificial
<400> 18
   Tyr Met Met Gly Asn Glu Leu Thr
   1 5
<210> 19
   <211> 4
   <212> PRT
   <213> Artificial
<400> 19
   Ser Ile Lys Ser
   1
<210> 20
   <211> 5
   <212> PRT
   <213> Artificial
<400> 20
   Ser Ile Lys Ser Leu
   1 5
<210> 21
   <211> 5
   <212> PRT
   <213> Artificial
<400> 21
   Val Ser Ile Lys Ser
   1 5
<210> 22
   <211> 6
   <212> PRT
   <213> Arrificial
<400> 22
   Ser Ile Lys Ser Leu Lys
   1 5
<210> 23
   <211> 7
   <212> PRT
   <213> Artificial
<400> 23
   Val Ser Ile Lys Ser Leu Lys
   1 5
<210> 24
   <211> 5
   <212> PRT
   <213> Artificial
<400> 24
   Ile Lys Ser Leu Lys
   1 5
<210> 25
   <211> 8
   <212> PRT
   <213> Artificial
<400> 25
   Lys Thr Lys Gly Ser Gly Asn Thr
   1 5
<210> 26
   <211> 4
   <212> PRT
   <213> Artificial
<400> 26
   Pro Gly Gln Asp
   1
<210> 27
   <211> 5
   <212> PRT
   <213> Artificial
<400> 27
   Pro Gly Gln Asp Cys
   1 5
<210> 28
   <211> 5
   <212> PRT
   <213> Artificial
<400> 28
   Gln Pro Gly Gln Asp
   1 5
<210> 29
   <211> 6
   <212> PRT
   <213> Artificial
<400> 29
   Pro Gly Gln Asp Cys Arg
   1 5
<210> 30
   <211> 7
   <212> PRT
   <213> Artificial
<400> 30
   Gln Pro Gly Gln Asp Cys Arg
   1 5
<210> 31
   <211> 5
   <212> PRT
   <213> Artificial
<400> 31
   Gly Gln Asp Cys Arg
   1 5
<210> 32
   <211> 9
   <212> PRT
   <213> Artificial
<400> 32
   Leu Ala Ala Phe Pro Glu Asp Arg Ser
   1 5
<210> 33
   <211> 321
   <212> DNA
   <213> Artificial
<400> 33
<210> 34
   <211> 107
   <212> PRT
   <213> Artificial
<400> 34
   Asp Ile Gln Thr Thr Gln Thr Thr Ser Ser Leu Ser Ala Ser Leu Gly
   1 5 10 15
   Asp Arg Val Thr Ile Ser Cys Arg Ala Gly Gln Asp Ile Ser Asn Tyr
   20 25 30
   Leu Asn Trp Tyr Gln Gln Lys Pro Asp Gly Thr Val Lys Leu Leu Ile
   35 40 45
   Tyr Tyr Thr Ser Arg Leu His Ser Gly Val Pro Ser Arg Phe Ser Gly
   50 55 60
   Ser Gly Ser Gly Thr Asp Tyr Ser Leu Thr Ile Ser Asn Leu Glu Gln
   65 70 75 80
   Glu Asp Ile Ala Thr Tyr Phe Cys Gln Gln Gly His Thr Leu Pro Trp
   85 90 95
   Thr Phe Gly Gly Gly Thr Lys Leu Glu Ile Lys
   100 105
<210> 35
   <211> 363
   <212> DNA
   <213> Artificial
<400> 35
<210> 36
   <211> 121
   <212> PRT
   <213> Artificial
<400> 36
   Asp Val Gln Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Asp
   20 25 30
   Tyr Ala Trp Asn Trp Ile Arg Gln Phe Pro Gly Asn Lys Leu Glu Trp
   35 40 45
   Met Gly Tyr Ile Arg Tyr Ser Gly Ser Thr Ser Tyr Asn Pro Ser Leu
   50 55 60
   Lys Ser Arg Ile Ser Ile Thr Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Ala Arg Asp Trp Pro Arg Pro Ser Tyr Trp Tyr Phe Asp Val Trp Gly
   100 105 110
   Ala Gly Thr Thr Val Thr Val Ser Ser
   115 120
<210> 37
   <211> 360
   <212> DNA
   <213> Artificial
<400> 37
<210> 38
   <211> 120
   <212> PRT
   <213> Artificial
<400> 38
   Gln Val Gln Leu Gln Gln Ser Gly Pro Glu Leu Val Lys Pro Gly Thr
   1 5 10 15
   Ser Val Arg Ile Ser Cys Glu Ala Ser Gly Tyr Thr Phe Thr Ser Tyr
   20 25 30
   Tyr Ile His Trp Val Lys Gln Arg Pro Gly Gln Gly Leu Glu Trp Ile
   35 40 45
   Gly Cys Ile Tyr Pro Gly Asn Val Asn Thr Asn Tyr Asn Glu Lys Phe
   50 55 60
   Lys Asp Lys Ala Thr Leu Ile Val Asp Thr Ser Ser Asn Thr Ala Tyr
   65 70 75 80
   Met Gln Leu Ser Arg Met Thr Ser Glu Asp Ser Ala Val Tyr Phe Cys
   85 90 95
   Thr Arg Ser His Tyr Gly Leu Asp Trp Asn Phe Asp Val Trp Gly Ala
   100 105 110
   Gly Thr Thr Val Thr Val Ser Ser
   115 120
<210> 39
   <211> 321
   <212> DNA
   <213> Artificial
<400> 39
<210> 40
   <211> 107
   <212> PRT
   <213> Artificial
<400> 40
   Asp Ile Gln Met Asn Gln Ser Pro Ser Ser Leu Ser Ala Ser Leu Gly
   1 5 10 15
   Asp Thr Ile Thr Ile Thr Cys His Ala Ser Gln Asn Ile Tyr Val Trp
   20 25 30
   Leu Asn Trp Tyr Gln Gln Lys Pro Gly Asn Ile Pro Lys Leu Leu Ile
   35 40 45
   Tyr Lys Ala Ser Asn Leu His Thr Gly Val Pro Ser Arg Phe Ser Gly
   50 55 60
   Ser Gly Ser Gly Thr Gly Phe Thr Leu Thr Ile Ser Ser Leu Gln Pro
   65 70 75 80
   Glu Asp Ile Ala Thr Tyr Tyr Cys Gln Gln Gly Gln Thr Tyr Pro Tyr
   85 90 95
   Thr Phe Gly Gly Gly Thr Lys Leu Glu Ile Lys
   100 105
<210> 41
   <211> 15
   <212> PRT
   <213> Artificial
<400> 41
   Gly Asn Tyr Ser Gln Gln Leu Gln Val Tyr Ser Lys Thr Gly Phe
   1 5 10 15
<210> 42
   <211> 15
   <212> PRT
   <213> Artificial
<400> 42
   Tyr Met Met Gly Asn Glu Leu Thr Phe Leu Asp Asp Ser Ile Cys
   1 5 10 15
<210> 43
   <211> 15
   <212> PRT
   <213> Artificial
<400> 43
   Lys Thr Lys Gly Ser Gly Asn Thr Val Ser Ile Lys Ser Leu Lys
   1 5 10 15
<210> 44
   <211> 16
   <212> PRT
   <213> Artificial
<400> 44
   Leu Ala Ala Phe Pro Glu Asp Arg Ser Gln Pro Gly Gln Asp Cys Arg
   1 5 10 15